# EUROPEAN PATENT SPECIFICATION

(11) **EP 3 463 154 B1**
(45) Date of publication and mention of the grant of the patent: **05.07.2023**
(21) Application number: 17737046.7
(22) Date of filing: 01.06.2017
(51) Int. Cl.: A61B 34/20, A61B 90/00, A61B 17/00

(54) **MOBILE SURGICAL NAVIGATION SYSTEM**
MOBILES CHIRURGISCHES NAVIGATIONSSYSTEM
SYSTÈME DE NAVIGATION CHIRURGICALE MOBILE

(30) Priority: 02.06.2016 US 201662344459 P
(43) Date of publication of application: 10.04.2019
(73) Proprietor: Atracsys Sàrl, 1070 Puidoux (CH)
(72) Inventor: MARTI, Gaëtan F., 1052 Le Mont-sur-Lausanne (CH); HÄLG, Maurice, 1073 Savigny (CH)
(74) Representative: Appleyard Lees IP LLP
(86) International application number: PCT/IB2017/053233
(87) International publication number: WO 2017/208186

(56) References cited:
- WO-A1-2015/048994
- DE-A1-102010 013 499
- US-A1- 2009 068 620
- US-A1- 2015 164 609
- Elektrotechniky Fakulta ET AL: "VYSOKÉ UCENÍ TECHNICKÉ V BRNE EVALUATION OF THE WI-FI TECHNIQUE FOR USE IN A NAVIGATED ORTHOPEDIC SURGERY", , 31 December 2012 (2012-12-31), pages 1-120, XP055918110, Retrieved from the Internet: URL:https://core.ac.uk/download/pdf/302985 31.pdf [retrieved on 2022-05-04]
- Revision: "Polaris Application Program Interface Guide", , 31 March 2011 (2011-03-31), pages 1-198, XP055918102, Retrieved from the Internet: URL:https://usermanual.wiki/Pdf/PolarisAPI Guide.378985072/view [retrieved on 2022-05-04]

## Description

### BACKGROUND OF THE INVENTION

The present invention relates generally to an optical tracking system for medical applications. In particular, it relates to a tracking system for Computer Assisted Surgery or Robotic Assisted Surgery. More specifically it relates to an optical tracking system for mobile surgical navigation with wireless communication means.

US 2009/068620 discloses a device for the contactless determination and measurement of a spatial position and/or spatial orientation of bodies using a tracking system, by means of which the bodies are located and brought into relation with one another, the tracking system, or at least components or modules thereof, being mobile.

US2015164609 relates generally to a navigation system that tracks objects in space by determining changes in the position and/or orientation of such objects over time. More specifically, it relates to navigation systems that utilize optical sensors and non-optical sensors to determine the position and/or orientation of objects.

The masters thesis of Jindrich Truhlár named "Evaluation of the Wi-Fi technique for use in a navigated orthopedic surgery", Brno University of Technology, Faculty of Electrical Engineering and Communication, Department of Biomedical Engineering, 2012; focuses on the use of wireless technologies in the OrthoPilot navigation system developed by B.Braun company.

### BRIEF SUMMARY OF THE INVENTION

The invention is defined in the appended claims and generally relates to a tracking system for mobile navigation which includes an Optical Tracking System 10 which is able to sense Fiducials 21 located on Markers 20 via Optical Sensors 11. The Optical Tracking System is compact enough to easily be installed in or nearby a surgical field (e.g. clamped on a Surgical Lamp, fixed on a surgical table via a mechanical arm or a pole, fixed on the separation of the anaesthetist, located on a surgical trolley, etc.). If the system is in the sterile field, it has either to be sterilisable or it is (at least partly) covered by a sterile cover.

The Optical Tracking System comprises processing means to compute from the raw data up to the pose (position + translation or position and/or orientation) of Markers 20 and transfer these data to a Tablet Computer 40 via Communication Means 30. These metrological data are finally used by a surgical application that can fit on a tablet PC. The surgical application may alternatively run within the Optical Tracking System and the Tablet just be a remote screen. Alternate sensors can be integrated within Markers. Marker sensor data can be transferred wirelessly to the Optical Tracking System and/or to the PC, so that there is a known relation between the optical pose or position and/or orientation data timestamp and the marker sensor data timestamp. This relation drastically simplifies data fusion for subsequent treatment and visualization.

There has thus been outlined, rather broadly, some of the features of the disclosure in order that the detailed description thereof may be better understood, and in order that the present contribution to the art may be better appreciated. Many additional features of the disclosure that will be described hereinafter in exemplary and non-limiting embodiments of the disclosure.

In this respect, before explaining several embodiments of the disclosure in detail, it is to be understood that the disclosure is not limited in its application to the details of construction or to the arrangements of the components set forth in the following description or illustrated in the drawings. The disclosure is capable of other embodiments and of being practiced and carried out in various ways. Also, it is to be understood that the phraseology and terminology employed herein are for the purpose of the description and should not be regarded as limiting. Other equivalent features may be considered in the scope and frame of the present disclosure.

An object and embodiment of the present disclosure is to provide an Optical Tracking System for Mobile Surgical Navigation that overcomes the need for a conventional navigation cart: instead of using a conventional computer located in the navigation cart, the optical tracking system directly communicates to a tablet preferably by wireless means. The tablet being able to provide both the processing needs, the display and the touch screen for the interaction of the medical team with the software.

Another object and embodiment of the present disclosure is to provide an Optical Tracking System for Mobile Surgical Navigation that communicates to the tablet and/or markers via one or several wireless links. The Optical Tracking System being preferably nomad and powered by a battery (or an accumulator).

Another object and embodiment of the present disclosure is to provide an Optical Tracking System for Mobile Surgical Navigation that provides a precise mechanism (e.g. hook(s), clip(s), magnet(s) or a combination therefrom) to fix a sterile cover / drape on the tracking system in order to provide a controlled optical path that can be used by the optical model to compensate on the distortions generate by the sterile cover / drape.

Another object and embodiment of the present disclosure is to provide a sterile drape fixation mechanism on the Optical Tracking System so that the drape and/or its fixation mechanism cannot be compensated but is designed to minimize the deterioration of the accuracy of the optical model.

Another object and embodiment of the present disclosure is to provide an Optical Tracking System for Mobile Surgical Navigation that provides a fixation mechanism to be easily attached to an equipment within or surrounding the surgical area.

Another object and embodiment of the present disclosure is to provide an Optical Tracking System for Mobile Surgical Navigation that offers a real-time supervision unit that is both working when operating or when stored. This unit enables to record temperature, hygrometry, shocks, vibration, time and/or GPS position in order to guarantee that the measurement precision / stability of the device is not affected by environmental hazards. When stored, the unit is operating thanks to an accumulator for example.

Another object and embodiment of the present disclosure is to provide an Optical Tracking System for Mobile Surgical Navigation that allows to record images / videos (respectively 3D depth images / videos) during the surgical intervention for archiving tele-conference, and/or aiming the system and/or patient monitoring purposes.

Another object and embodiment of the present disclosure is to provide an Optical Tracking System for Mobile Surgical Navigation that uses real-time optical sensor data (e.g. disparity images) for Surgical Human-computer Interfaces like to capture 3D movements of the surgeon's hands and use them as an input device for the surgical Software application to interact with the system (e.g. in a similar way the Microsoft Kinect is used to interact with the Microsoft Xbox). The same concept could be used to record patient's movements in a rehabilitation configuration.

Another object and embodiment of the present disclosure is to use a wireless communication means with a deterministic packet transmission between Markers and Optical Tracking System. Normally, existing wireless technology standards either cannot provide real-time guarantee on packet delivery or are not fast enough to support sensor fusion or high-speed control systems which typically require high sampling rate and low latency. Such nondeterministic packet transmission and insufficiently high sampling rate will severely hurt the sensor fusion (respectively control) performance. Therefore, in the context of the present disclosure, the notion of deterministic packet transmission is to be understood as meaning ensuring a deterministic or determined timing guarantee on packet delivery and high sampling rate up to the kHz range. The overall timing and transmission times or delays are determined or known and taken into account in the method to obtain this deterministic approach.

Further, as in the present disclosure, the Wireless sensor network is generally used to sense and capture information about physical events, from which correlation patterns are then extracted collaboratively, to this end, sensors must be time synchronized to project the relative chronological order of occurrences in an event.

This wireless communication means enables to synchronize optical pose data or position and/or orientation data with sensor data generated by the marker such as metrological data (e.g. gyroscopic, inertial, magnetometer, magnetic, ultrasound, etc.), environmental data (e.g. temperature) or patient's data (e.g. heart rate, blood pressure, electro-cardiograms, electrodes, - deep brain - stimulators, etc.).

Another object and embodiment of the present disclosure is to provide an Optical Tracking System for Mobile Surgical Navigation that have the navigation application embedded in the Optical Tracking System. In this configuration, a Tablet Computer is no longer necessary. Interface with the surgeon could be achieved with LEDs and/or alternate outputs on the markers, internal buzzer, and/or an external screen in the OR not initially dedicated to navigation. This object allows to avoid an external computer (respectively a touch screen) to be used during the intervention. Navigation interface can be placed partly or exclusively on the surgical tools.

Another object and embodiment of the present disclosure is to synchronize active Markers and/or Optical Tracking Systems with other sensors in the OR by mean of wireless Communication Means enabling to have a distributed wireless network of Optical Tracking Systems and Markers around the patient that is operating with a common timestamp. This is especially interesting for synchronization or sensor fusion.

Other objects, advantages and embodiments of the present disclosure will become obvious to the reader and it is intended that these objects and advantages are within the scope of the present disclosure. To the accomplishment of the above and related objects, this disclosure may be embodied in the form illustrated in the accompanying drawings, attention being called to the fact, however, that the drawings are illustrative only and not to be construed in a limiting manner, and that changes may be made in the specific construction illustrated and described within the scope of the present application.

In an embodiment, the disclosure concerns an optical tracking system for medical applications, comprising an optical tracking unit with at least one sensor, a processing unit (such as a microprocessor) and energy providing means, wherein the optical tracking unit, the sensor and the processing unit are being integrated in a single housing, and wherein the system further comprises at least one marker to be attached to an object or to a person, a separate and/or remote display means receiving data at least from the tracking unit; and wireless communication means connecting at least the tracking unit with the display means and allowing data to be transmitted between the tracking unit and the display means, wherein the sensor detect the marker(s) so that the position and/or orientation of the marker relatively to the tracking unit is determined by a data processing of sensor data, and wherein the data processing is carried out by the processing unit in such a way that the processed data transmitted by the wireless communication means between the tracking unit and the display means is optimized to the communication channel. The optimization may be for example a reduced or compressed size of data, or adaptation to bandwidth etc. as described in the present specification.

In an embodiment, the display means is a tablet computer or a PC, or a screen or another device as described herein or equivalent.

In an embodiment, the energy providing means comprises a battery pack, and the battery pack is integrated in the same housing as the optical tracking unit.

In another embodiment, the energy providing means is a battery pack and the battery pack is attached to the housing of the system and can easily be removed and/or replaced for example by another pack.

The energy providing means may also be an accumulator for example or a combination of battery pack and accumulator.

In an embodiment, the tracking unit comprises at least two sensors, for example cameras. Other equivalent optical sensors may be envisaged as well.

In an embodiment, triangulation means are used to retrieve the 3D positions of the fiducials.

In an embodiment, at least one of the sensor is a camera.

In an embodiment, the at least one marker comprises fiducials. The fiducials are preferably made of light generating elements or comprise such elements.

In an embodiment, the light generating elements are both used to transmit data and used as fiducials during the sensing phase.

In an embodiment, the tracking unit comprises attachment means to attach a surgical drape. The attachment means may be hook(s), clip(s), magnet(s), glue, Velcro^{™} and equivalent, (dual-face) tape etc.

In an embodiment, wireless communication means comprise for example Wi-Fi, IEEE 802.15.1 or Li-Fi or other similar communication networks.

In an embodiment, the present disclosure concerns a surgical device combined with an optical tracking system as defined in the present description. Preferably, the tracking system is a portable system with portable parts and/or elements forming the system according the features of the disclosure described herein.

In an embodiment, the disclosure concerns an optical tracking method for medical applications, comprising the following steps
- ) at least one marker placed on an object or on a person and detected by at least one sensor of a tracking unit, said sensor providing sensor data;
- ) the position and/or orientation of the marker detected by the sensor is at least determined by data processing of the sensor data in a processing unit of the tracking unit;
- ) the determined position and/or orientation is transmitted as processed data from the tracking unit to a display unit using a wireless communication through wireless communication means, wherein the processed data is optimized for said wireless communication and communication channel.

In an embodiment of the method, the optimization of data may be a reduction of data size or optimization of bandwidth or frequency transmission.

In an embodiment of the method, the position and/or orientation of the marker is determined by using marker data provided by the marker(s). The marker data may be combined with the sensor data or replace the sensor data as described in embodiments of the present disclosure detailed herein.

In an embodiment, the disclosure concerns an optical tracking system for medical applications, with an optical tracking unit comprising at least one sensor, a processing unit with a processing unit clock and energy providing means,
wherein the system further comprises at least one marker to be attached to an object or to a person;
wherein said marker comprises at least one marker sensor generating marker data and having a marker sensor clock, wireless communication means connecting at least the tracking unit with the marker and allowing data being transmitted between the tracking unit and the marker, wherein the sensor(s) detect(s) the marker(s) so that the position and/or orientation of the marker(s) relatively to the tracking unit is determined by a data processing of sensor data and/or of marker data,
wherein during the data processing the data timestamps are synchronized in real time to compensate the difference in time between the clocks.

In an embodiment, the data, for example the maker data and/or the sensor data and/or the position and/or orientation are transmitted to a display means. The display means may be a tablet computer or a PC, or a screen or another device as described herein or equivalent. The transmission may be wireless or not.

In an embodiment, the time difference of clocks between the sensor data and its related marker data is determined.

In an embodiment, the marker sensor comprises data from a three-axis accelerometer and/or a three-axis gyroscope that is part of the marker data.

In an embodiment, the synchronization timestamp is estimated by correlation of common physical signals that can be retrieved independently from optical data and sensor data.

In an embodiment, the physical signals used in the system and method according to the disclosure are acceleration and/or velocity and/or position.

In an embodiment, sensor data or marker data can be either interpolated or extrapolated to fit a common timestamp.

In an embodiment, the markers comprise fiducials in the shape of light generating elements.

In an embodiment, the light generating elements are both used to transmit data during wireless communication and used as fiducials during a sensing phase.

In an embodiment, the wireless communication means is a deterministic packet transmission technology.

In an embodiment, the wireless communication means is optical or near-infrared communication.

In an embodiment, the deterministic wireless packet transmission is using light generating elements that are also used as fiducials during the sensing phase.

In an embodiment, the at least one marker further comprises a data bus, wherein said tracking unit clock is transferred to said marker via the synchronized/deterministic wireless communication means and tracking unit clock is further transferred on the data bus.

In an embodiment, the marker the position and/or orientation provided by the optical tracking unit and marker sensor data are fused to provide an improved marker the position and/or orientation result.

In an embodiment, marker the position and/or orientation provided by the optical tracking unit and marker sensor data are fused to provide a marker the position and/or orientation at a higher update rate than the speed of the optical sensor.

In an embodiment, the sensor is a camera. The unit also preferably comprises at least two sensors, for example cameras. Other equivalent optical sensors may be used as well.

In an embodiment, triangulation means are used to retrieve the 3D positions of the fiducials.

In an embodiment, for example in all realizations, the tracking system is preferably portable.

In an embodiment, the disclosure concerns a surgical device combined with an optical tracking system as defined in the present specification.

In an embodiment, the disclosure concerns an optical tracking method for medical applications, comprising the following steps
- ) at least one marker placed on an object or on a person is detected by at least one sensor of a tracking unit, the sensor providing sensor data with a sensor timestamp;
- ) wherein the marker comprises at least a marker sensor generating marker data with a marker sensor clock and a marker timestamp;
- ) position and/or orientation of the marker detected by the sensor is at least determined by data processing of the sensor data and/or marker data in a processing unit of the tracking unit, the processing unit having a processing unit clock;
wherein during the data processing the timestamps are synchronized in real time to compensate the difference in time between said clocks.

In an embodiment, the determined position and/or orientation is transmitted as processed data from the tracking unit to a display means. This can be done for example using a wireless communication through wireless communication means as described herein or cables/wires.

In an embodiment, the time difference between sensor data and its related marker data is determined.

In an embodiment, the sensor data comprise data from a three-axis accelerometer and/or a three-axis gyroscope.

In an embodiment, sensor data or marker data are either interpolated or extrapolated to fit a common timestamp.

In an embodiment, the at least one marker is an optical marker providing an optical signal to be detected by the sensor.

In an embodiment, marker position and/or orientation provided by the optical tracking unit and marker data are fused to provide an improved marker position and/or orientation.

In an embodiment of the method marker the position and/or orientation provided by the optical tracking unit and accelerometer and/or gyroscopic marker data are fused to provide an improved marker the position and/or orientation.

In an embodiment, the present disclosure concerns a method of using an Intelligent Tool, such as a robot, comprising at least a tracking system as defined in the present application, where at least one Marker is located on the tool and another one on the patient wherein the tool uses a deterministic wireless communication and the position and/or orientation data and/or additional data coming from the different connected elements to perform a predetermined specific task.

In an embodiment, the tool stops operating if a specific region is reached, the region being defined on the base of pre- or intra-operative images. The region may be for example a part of the body of the patient.

In an embodiment of the method, the tool receives in real-time via deterministic wireless means the transformation between the marker located on the tool and the one located on the patient and use it to operate.

In an embodiment of the method, the tool receives in real-time via deterministic wireless means the position and/or orientation of the marker located on the tool and the marker located on the patient and use these data to operate.

Preferably, in all embodiments of the device or the method as defined herein, the wireless communication is done via a physical layer based on the IEEE 802.11 or IEEE 802.15 standards.

### BRIEF DESCRIPTION OF THE DRAWINGS

Various other objects, features and attendant advantages of the present disclosure will become fully appreciated as the same becomes better understood when considered in conjunction with the accompanying drawings, in which like reference characters designate the same or similar parts throughout the several views, and wherein:
**FIGURE 1** illustrates the main elements of the invention in a schematical perspective view;
**FIGURE 2** presents a traditional Navigation Cart used in medical tracking applications;
**FIGURE 3** illustrates an embodiment of the present disclosure;
**FIGURE 4** illustrates an embodiment of the present disclosure;
FIGURE 5 illustrates a sequence diagram of an optical tracking system using active markers according to an embodiment of the present disclosure;
FIGURE 6 illustrates an embodiment of the invention;
FIGURE 7 illustrates a sequence diagram of an optical tracking system using active markers according to an embodiment of the present disclosure;
FIGURE 8 illustrates a sequence diagram of an optical tracking system using active markers according to an embodiment of the present disclosure;
FIGURE 9 illustrates an embodiment that correspond to the sequence diagram of Figure 8 (with only one marker);
**FIGURE 10** illustrates an embodiment that correspond to the sequence diagram of Figure 7 (with only one marker).
**FIGURE 11** illustrates an embodiment of the disclosure without display means.

### DETAILED DESCRIPTION OF THE INVENTION

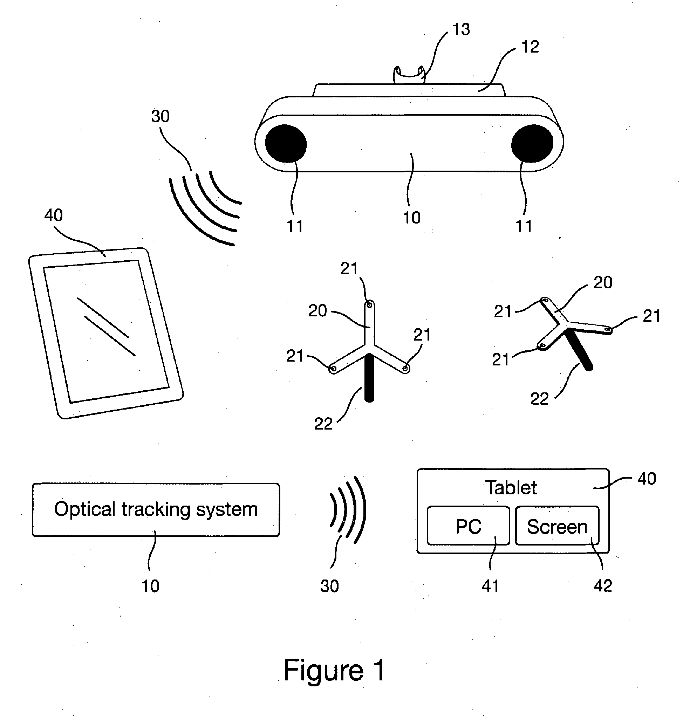

Figure 1 illustrates schematically main elements of an embodiment of the invention. The setup comprises an Optical Tracking System 10, in this example comprising two cameras 11, a battery pack 12 and a fixation mechanism 13. Markers 20 comprising fiducials 21 are fixed 22 to the patient and/or the surgical instruments. Fiducials 21 are identified by the camera, and triangulated to get their 3D position. Pose (orientation and/or position) of the markers 20 can be determined if at least three fiducials 21 are seen by the two cameras and triangulated. Poses (and other data) can be transferred - in this example via a wireless link 30 - to a tablet 40. The tablet comprises for example a PC / processing unit 41, a touch-screen 42 and means to receive the data from the tracking system via the link 30. The Optical Tracking System 10, as well as the tablet 40 can be draped to be operated within the sterile field or otherwise protected.

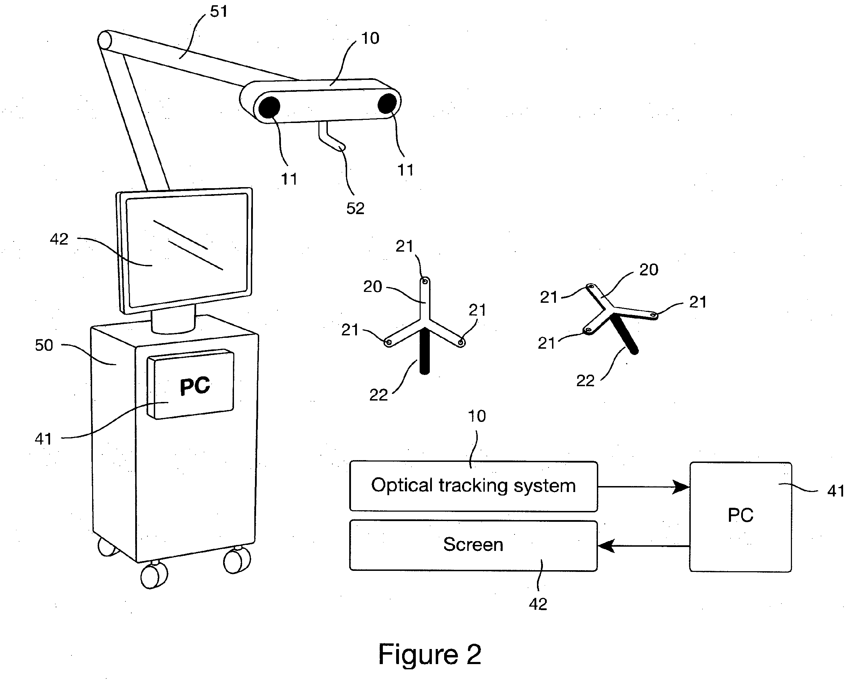

Figure 2 illustrates a traditional Navigation Cart 50. The Optical Tracking System 10, the Markers 20 and their sub-components are similar to Figure 1 except that the Optical Tracking System 10 is on a mechanical arm 51 (resp. pole) fixed on the cart. This arm contains necessary cables for the power supply and to connect the tracking system directly to the PC or computer 41. The tracking system may be oriented to aim the Markers 20 using a Handle 52. The cart is powered by a cable directly plugged into the wall outlet. The navigation application runs on the PC 41 and is displayed on a (touch) screen 42. The problems linked to a traditional Navigation Cart 50 are the following: Navigation Carts 50 are bulky and cumbersome and require consequence storage space; There are numerous risks of damage of the Optical Tracking System 10 when moving in/out of the storage space (e. g. Optical Tracking System got damaged when hit through a door, damages due to vibrations of the cart when rolled on a paved floor); There are line of sight (occlusions) problems as Markers 20 are located inside the sterile field, the Optical Tracking System 10 is placed outside and nurses / surgeons are moving in-between.

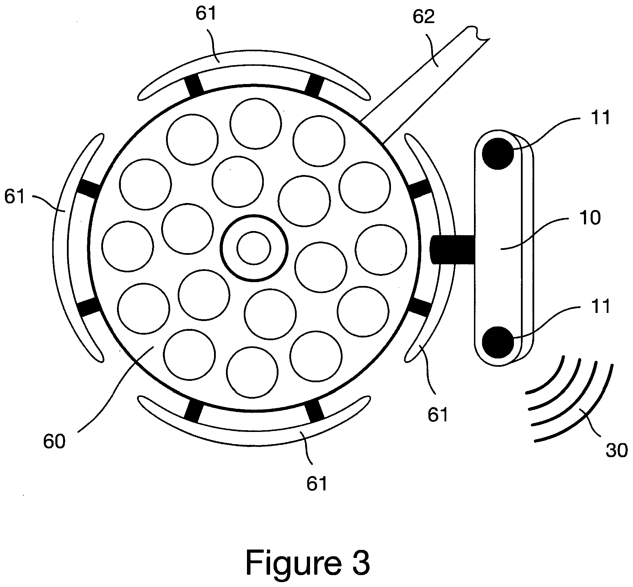

Figure 3 illustrates another embodiment of the present disclosure. The Optical Tracking System 10 is clipped on a (non-sterile) Holder 61 of a Surgical Lamp 60. The Surgical Lamp 60 being fixed to the ceiling via a Mechanical Arm 62. Power for the Optical Tracking System 10 is either taken from the lamp or embedded in a battery pack (not represented here but presented in figure 1). The Optical Tracking System transfers data to the Tablet by means of a wireless communication 30. Wireless data transfer is optimized to fit transmission bandwidth while delivering the maximum update rate with the minimum latency to the (tablet) PC.

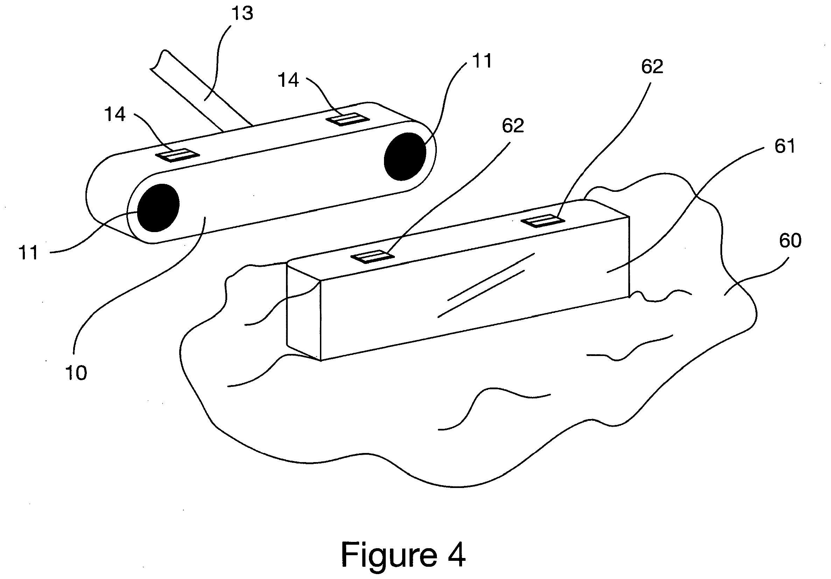

Figure 4 illustrates an alternative embodiment of the present disclosure. The Optical Tracking System 10 comprises a Mechanism 14 to precisely fix a Sterile Window 61 in front of the Optical Sensors 11. The Sterile Window 61 is preferably integrated in a Sterile Drape 60 that covers partly or entirely the Optical Tracking System 10. The Sterile Window 61 and/or the Sterile Drape 60 has a fixation mechanism (62 - e. g. hook, clip, magnet, or other equivalent etc.) to precisely attach the window on another part that is located on the Optical Tracking System 14. Ideally the window should fit parallel to the Optical Sensors 11 at a known distance. This precise fixation enables to adapt the optical model of the tracking system 10 to compensate for optical diffractions through the Sterile Window 61.

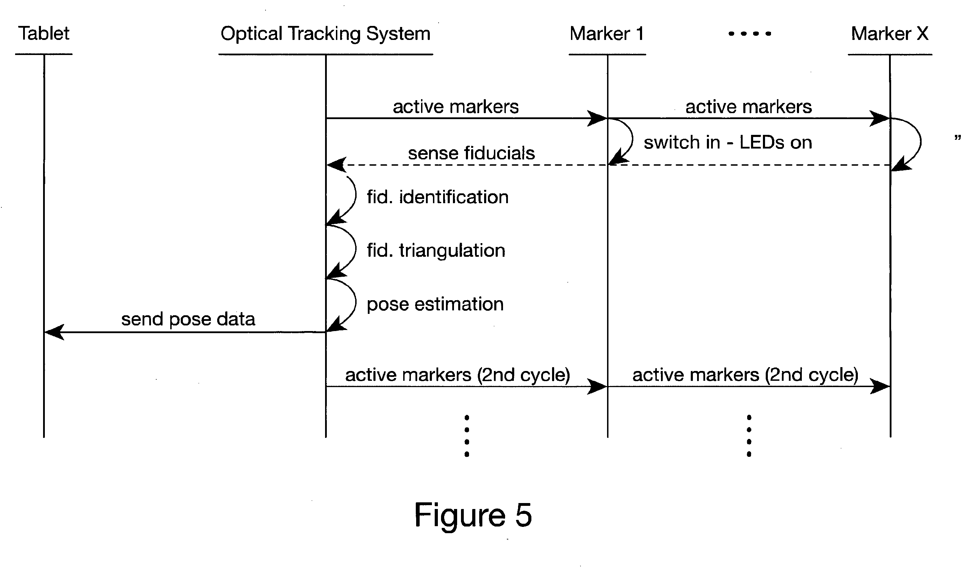

Figure 5 illustrates a typical sequence diagram of an Optical Tracking System using active Markers with a deterministic wireless packet transmission using infrared light. Data transfer between the Optical Tracking System 10 to the Tablet PC 40 can either be wired or wireless. The tracking system queries the Markers 20 to activate them ("active markers"). The Markers 20 switch their IR-LEDs 21 on ("switch IR-LEDs on") as fiducials. The fiducials (IR-LEDs 21) are simultaneously detected by the Optical Tracking System ("sense fiducials"). Fiducials 21 are further identified ("fids. identification") within data generated by the different Optical Sensors (e.g. images if the optical sensor is a camera) and triangulated ("fids. triangulation"). Pose (position and/or orientation) of the markers 20 is estimated in the tracking system ("pose estimation") and further send to the Tablet ("send pose data"). A subsequent cycle starts with the same sequence ("...") for tracking purposes.

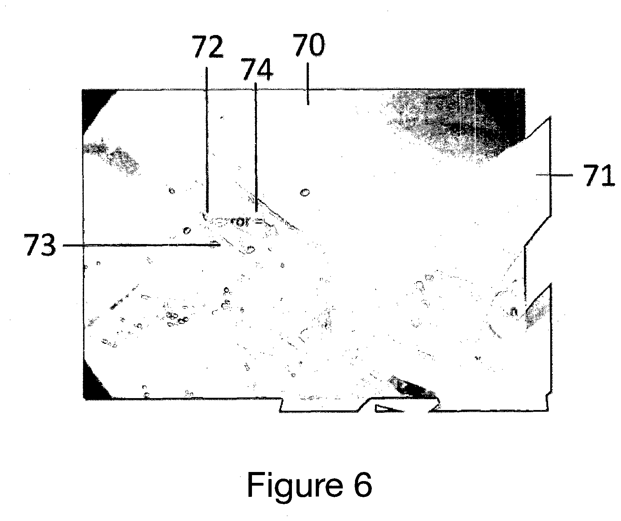

Figure 6 illustrates another embodiment of the disclosure. The Optical Tracking System 10 is used for both tracking and overlaying information on camera images 70 either in real-time and/or for archiving purposes after a medical, for example surgical, intervention. Different overlays are possible as 72 refers to the tooltip position of a surgical instrument in the image (given the instrument is tracked and the position of the tooltip is known with respect to the Marker coordinate system). Reference 71 presents an augmented-reality view (overlay of a preoperative 3D reconstruction of the patient in the optical sensor image). Reference 73 presents the 3D trajectory of the tool (and optionally the distance to the patient). Reference 74 presents the Marker registration error of the tool. Any other type of information could be overlaid in the camera images like an ultrasound probe image, cardiogram, heartbeat, etc.

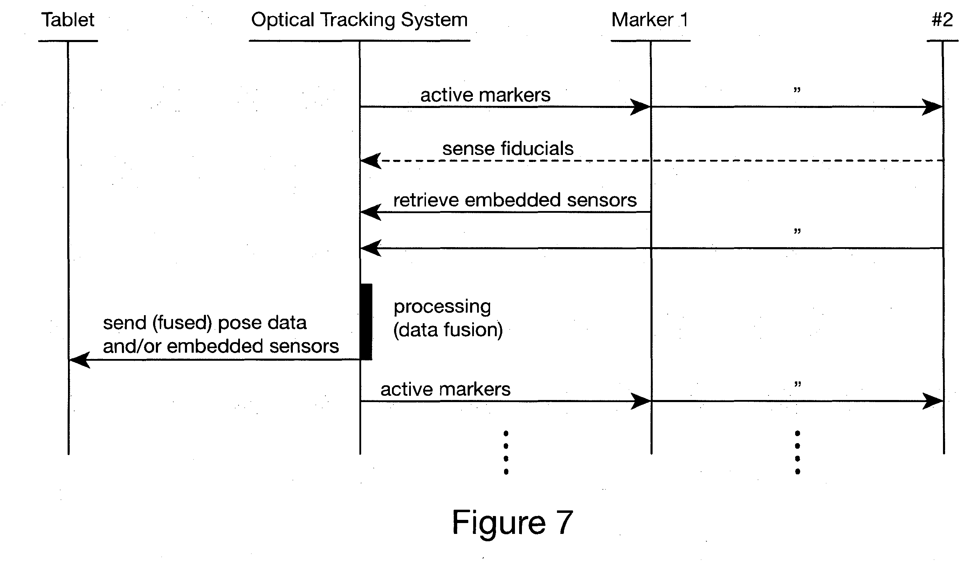

Figure 7 illustrates a sequence diagram of an Optical Tracking System using active Markers 20, which have embedded sensors. The sequence is similar to Figure 5 except that there is a wireless link with deterministic wireless packet transmission between the Markers 20 and the Optical Tracking System 10 to transfer the embedded sensors data linked to an optical acquisition to the Optical Tracking System ("retrieve embedded sensors"). The embedded sensor data can be used alone or together with the optical data to estimate the marker pose, e.g. position and/or orientation, (processing - data fusion). The advantage of having a deterministic wireless packet transmission is the possibility to have both the pose (position and/or orientation) data and the embedded sensors marker data with a common timestamp as all the timings within the system are deterministic. The resulting pose (position and/or orientation) and/or the embedded sensor data are finally send to the tablet ("send - fused - pose data and/or embedded sensors"). Note that several embedded sensors data packets can be send during an optical acquisition cycle, dependent on the speed/frequency acquisition of data from both sources (embedded sensors data and optical data). This method can be used to have a common timestamp to compute a sensor fusion between optical 3D/6D data and embedded marker pose (position and/or orientation) data. This method is useful for example to augment the precision of the measurements or to compensate data loss. A loss can typically occur when the optical detection is impaired, when a marker 20 cannot be detected by the sensors 11. This can happen if somebody stands in the way (for example the surgeon) or another obstacle is present and, during a certain time, the optical detection is not working properly. The missing optical data may thus be replaced by the embedded sensor data.

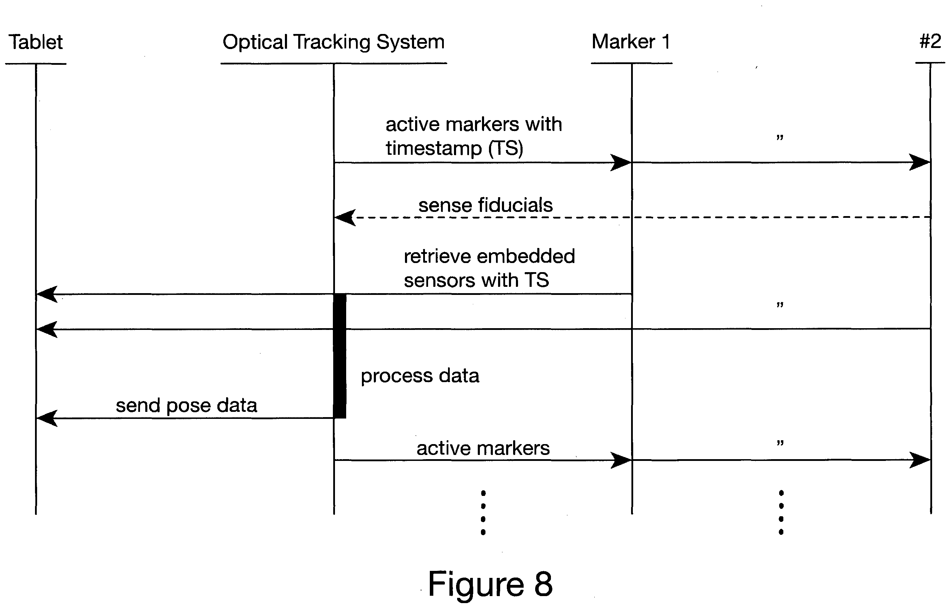

Figure 8 is an example of a sequence diagram of an Optical Tracking System 10 using active Markers 20. The active Markers having embedded sensors. This is an alternative sequence to figure 7. During the active markers message, the timestamp and/or any other information related to the current acquisition cycle of the tracking system is sent to the markers ("active markers with timestamp - TS"). The markers directly transmit embedded sensors information to the tablet including a timestamp information ("retrieve embedded sensors with TS") with any wired or wireless transmission means. The Optical Tracking System 10 also send the pose (position and/or orientation) data including the timestamp information to the tablet ("send pose data") with any wired or wireless transmission means. This method allows to resynchronize the timestamps of data coming both from the Markers and from the Optical Tracking System. Note that the transmission delays between the various components is ideally deterministic, which enables optimal sensor fusion directly on the tablet.

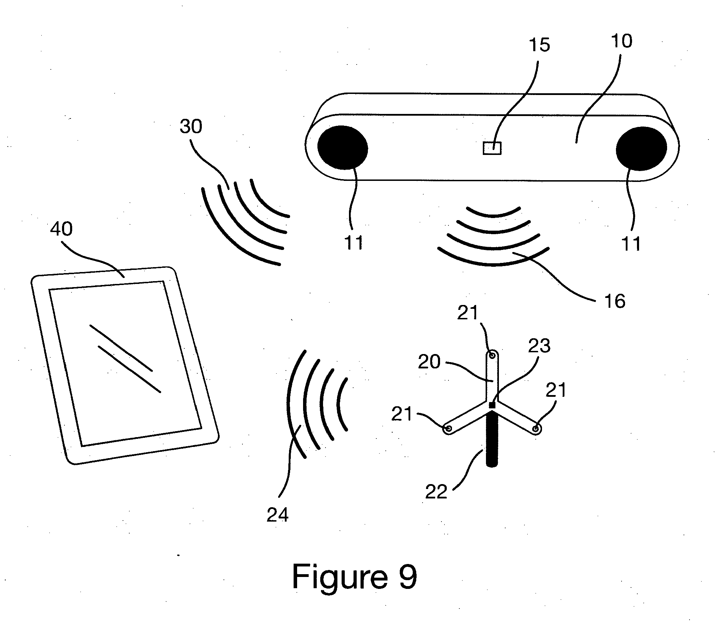

Figure 9 illustrates concrete embodiment that correspond to the sequence diagram of Figure 8 (with only one marker). In this example, the Optical Tracking System 10 further comprises a real-time deterministic wireless packet transmission 15 that is used to transmit the timestamp of the Optical Tracking System, as well as to in-form the markers when the optical measurement is taking place 16. If the Fiducials 21 are light elements, this piece of information corresponds to the moment when the lights of the Marker are switched on. Both the transferred timestamp of the optical tracking system and the deterministic transmission time enable to synchronize the internal clock of the marker with the clock of the optical tracking system. The Marker 20 further comprises Embedded Sensors that acquire data with the synchronized clock 23 and means to transfer the data 24 to the Tablet PC 40. Optical tracking data are transferred to the Tablet PC via another transmission means 30. As both tracking data and embedded sensor data are acquired on the same synchronized clock, it is possible to operate precise data fusion on the Tablet PC.

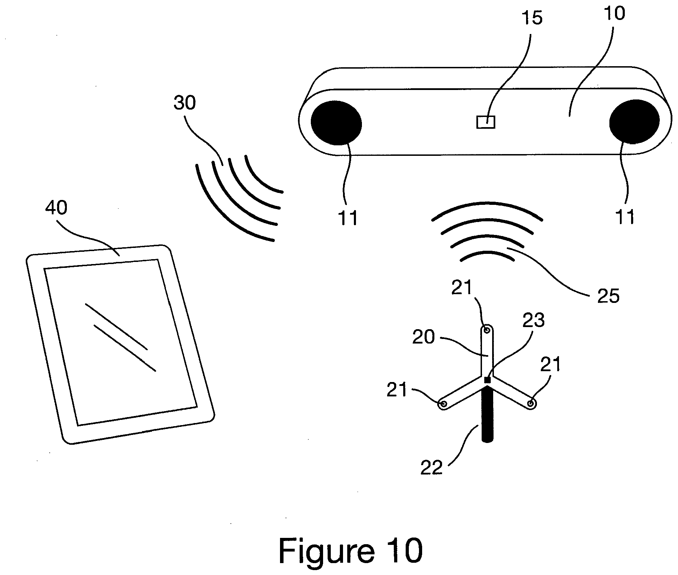

Figure 10 illustrates an embodiment that corresponds to sequence diagram of Figure 7 (with only one marker). In this example, the Optical Tracking System 10 further comprises a real-time deterministic wireless packet receptor 15 that is used to retrieve the markers embedded sensor 23 data 25. As the system is deterministic, the Optical Tracking System knows the exact time when the marker sensors data has been acquired, which enable to fuse optical data with data from embedded marker sensors. Data can be fused within the tracking system and further transmitted 30 to the Tablet PC 40. Raw optical tracking data and/or embedded sensor data with a synchronized timestamp can alternatively be transmitted to the Tablet PC 40.

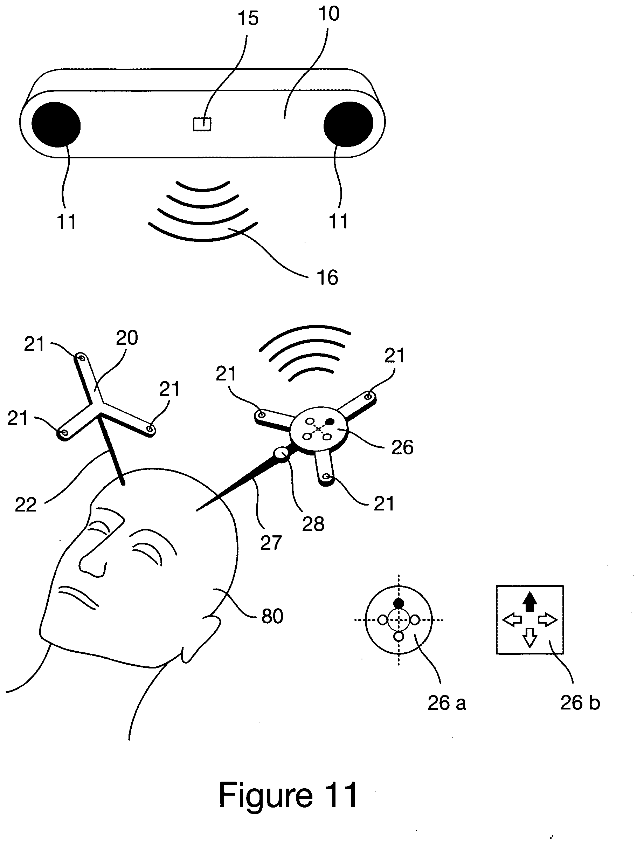

Figure 11 illustrates an embodiment of the disclosure without display means. The setup comprises an Optical Tracking System 10 that comprises a navigation Software for craniotomies as an application example. A marker 20 is fixed on the skull of a patient 80 via an attachment mechanism 22 (for example a screw or another equivalent means). The surgeon is holding a probe 27 that is a basically marker with a tooltip. The probe has an aiming interface 26 composed if a LCD screen 26b or a LED arrangement 26a. The Optical Tracking System can communicate data to the Markers via wireless communication means 16. Transferred data can comprise information on how to update the aiming interface. Prior to the craniotomy, the surgeon defines the ablation area based on imaging data. After a registration process, the aiming system 26 on the probe 27 helps the surgeon to move (and optionally orient) the tooltip to the correct location on the skull surface to match the planning. For example, the upper LED may blink to indicate that the probe should be moved upwards. Optional buttons 28 on the probe may be used to interact with the navigation Software (e.g. capture surface points during the registration process or move to the next ablation zone).

### Overview

Turning now descriptively to the drawings, in which similar reference characters denote similar elements throughout the several views, the figures illustrate embodiments of an Optical Tracking System 10 which is able to sense Fiducials 21 located on Markers 20 via Optical Sensors 11. The Optical Tracking System has processing means in the same housing to compute the pose (position + translation) of the Markers 20 and transfer them to a Tablet, computer 40 via Wireless Communication Means 30. These metrological data are finally used by a surgical application.

In an embodiment, sensors may be integrated within the Markers 20. Marker sensor data generated by said integrated sensors may be transferred wirelessly to the Optical Tracking System 10 and/or to the PC 40, so that there is a known relation between the optical pose (position and/or orientation) data timestamp (linked to the optical detection with the sensors 11 and the markers 20) and the marker sensor data timestamp (linked to the data generated by the integrated sensors). This relation drastically simplifies data fusion of the marker data sensor with the optical tracking data.

### Optical Tracking System

An Optical Tracking System 10 comprises one or several sensors further called Optical Sensors 11. Optical Sensors 11 retrieve angular information of the Fiducials 21 in view. Fiducials 21 are then identified by cross-checking them on the different Optical Sensors 11. Triangulation is used to compute the 3D positions of the Fiducials 21. If at least three Fiducials 21 are affixed together on one Marker 20, it is possible to compute its pose (that is its position and/or orientation).

Fiducials 21 may either be active: they transmit light (e. g. LEDs) or passive: they define a specific pattern (e. g. QR Codes) or they reflect light (e. g. Reflective spheres, disks). Most of Optical Tracking Systems are operating in the visible spectrum or near infrared (IR). Tracking systems using reflective material usually have rings of IR-LEDs around the Optical Sensors 11. These IR-LEDs are flashed, light is reflected on the reflective material and finally captured by optical sensors (e. g. Cameras).

The Optical Tracking System 10 can be powered via wire (e. g. USB charger) or alternatively by mean of a battery pack 12. Battery pack may be recharged by a conventional external charger or by an internal charging electronics. Other charging mechanisms may be considered like solar cells, induction etc.

Depending upon the position of the Fiducials 21 and the Optical Sensors 11, triangulation-based optical trackers can be divided into two categories: the inside-out and the outside-in systems. Inside-out systems place the Fiducials 21 at fixed places in the environment and the Optical Sensors 11 on a target object. On the other hand, outside-in systems place the Fiducials 21 on the target object and the Optical Sensors 11 at fixed places in the environment.

A fixation mechanism 13 allows to fix the system on an inside-out configuration like on a traditional Navigation Cart 50. More innovative locations could be on a pole or an arm (fixed on the operating table, on the separation between the anaesthetist and the surgical field, or on the cluster of surgical lamps), clipped directly on a surgical lamp (see Figure 3) or other equipment (e. g. C-Arm, CT, MRI, surgical microscope). One could imagine an add-on handler so a surgeon/nurse could hold the device when needed (e.g. Naviswiss Clip-on style). The tracking system could even be fixed directly on the surgeon (e. g. Integrated in the surgical helmet, placed on glasses, etc.). In an outside-in configuration, it can be fixed on a surgical drill, on an endoscope, on the patient (e. g. via an orthopaedic pin - Steinmann nail). If the tracking system is operating in the sterile field, it should be (at least partly) wrapped with a sterile drape 60. Care should be taken to have the Optical Sensors 11 as less perturbed as possible with this drape as light will be reflected and will bias the triangulation. An optical-grade window 61 is typically inserted in the sterile drape to reduce these perturbations and improve the overall accuracy of the system. If the placement of the optical windows of the surgical drape is reproducible, it is possible to integrate the diffraction of the light within the optical model of the system. Taking into account this windows will reduce the triangulation error and provide a better overall accuracy of the system. A straight forward example would be to calibrate the optical tracking system with the reproducible drape in front of it. If it cannot be compensated, the drape should be designed to reduce as much as possible the optical degradations.

Optical Sensors 11 are any types of cameras including conventional CMOS/CCD array sensor, light field camera, any optical system composed of a sensor and either a diaphragm and lens(es) (e.g. a conventional digital camera system) or simply a mask pattern in front of the camera sensor. 2D/3D SpaceCoders technology is an alternate possibility ("spaceCoder: a Nanometric 3D Position Sensing Device", author E. Grenet & al, published in CSEM Scientific & Technical Report 2011). A single camera can be used if the markers comprises at least four fiducials. The company Intellijoint has designed such a camera as an example.

Near infrared filter can be used in combination with infrared LEDs. More generally, any filter can be used to increase the SNR.

Communication Means 30 to the PC are preferably wireless communication means such as Wi-Fi, wireless personal area network (WPAN), ANT+, Li-Fi, NFC, etc. It can alternatively be any wired connection (Ethernet, USB, Firewire, etc).

Communication with active Markers can be realized by IR uni- or bi-directional optical communication or any wireless Communication Means already presented. A real-time deterministic wireless transmission is preferred.

Figures 7 and 8 present two configurations where such wireless communication enables to retrieve embedded sensors with a common timestamp. Such approaches allow precise sensor fusions techniques. For example, if the markers are equipped with 3D accelerometers and 3D gyroscopes, it is possible to fuse the optical pose (position and/or orientation) with the marker sensor data as presented in ("An Inertial and Optical Sensor Fusion Approach for Six Degree-of-Freedom Pose Estimation", Changyu & al, published in Sensors 2015). In this last example, the key aspect is to use a deterministic wireless transmission so that it is possible to have a common timestamp between the data provided by the different sources. A difference of a few microseconds can drastically bias the sensor fusion. Note that wireless protocols like UDP or TCP-IP over Bluetooth or Wi-Fi cannot guarantee a deterministic transmission. As such, real-time inertial and optical sensor fusion cannot be achieved using such non-deterministic technologies (see RT-WiFi: Real-Time High-Speed Communication Protocol for Wireless Cyber-Physical Control Applications, Yi-Hung Wei & al, Real-Time Systems Symposium (RTSS), 2013 IEEE 34th).

The device can be powered by a wire which can also serve to transfer data (e.g. PoE, USB, Firewire). A data cable can be only used to power the device, data being effectively transferred to the PC wirelessly with any suitable protocol (for example as mentioned above). Device can be powered by mean of a battery, electrolytic capacitor, supercapacitor and/or by induction or other equivalent.

In order to design a nomad optical tracking system, the optical sensors, computation up the 3D position of fiducials (respectively pose or position and/or orientation of markers) and the power supply should ideally be integrated in the same housing. Preferably, it should be possible to replace the battery during the surgery (e.g. via a clipping, magnetic mechanism or other attachment means) and the wireless transmission should be designed for a low-energy transmission. It practically means that the quantity of data to transmit should be optimized for the used wireless packet transmission technology and ideally as small as possible. Thus, the transmission time will be as short as possible and also the energy use will be optimized.

Fixation can be permanent (screws) or easily removable using a clipping mechanism (magnets, clips, etc.) so that the tracking system 10 may rapidly be attached/detached.

The system 10 can be designed to offer a fixation mechanism (magnets, clips, notch, hole, etc.) to perfectly place the optical-window of the sterile drape. The attachment mechanism should be designed so that whatever drape is placed, the light going thought the optical window is behaving similarly. The optical model for triangulation is of course preferably designed to take this optical window into account.

Near infrared optical tracking systems are widely used in surgery. They basically comprise a set of cameras with a known baseline (respective transformation between the cameras). The cameras operate in the near infrared so that flashes are not disturbing the users. The flashes are emitted by infrared lights that are preferably arranged on a ring around the camera lenses and/or by IR-LEDs (fiducials) located on the Markers.

Most frequently used cameras are stereo-camera (comprising two cameras) fixed on a bar. The respective pose or position and/or orientation of the cameras is factory calibrated. Once a fiducial is identified by both cameras, it is possible to compute its 3D position by triangulation. System comprising more cameras are also possible. In this case, triangulation is carried out using the data coming from the different optical sensors.

Example of commonly used passive optical stereo-tracking systems which are commercially available are Atracsys^{™} infiniTrack^{™}, fusionTrack^{™} and spryTrack^{™}, NDI Polaris^{™} or Axios CamBar^{™}.

Mono-camera systems can alternatively be used. They use projective geometry and at least 4 points on a marker to compute the 3D position of the fiducials. An example of such camera is the one developed by the company named Intelijoint^{™}.

Tag-based stereo cameras could alternatively also used both in the visible and the near infrared. These cameras detect specific patterns (tags) that could be for example QR-Codes and use elements of the pattern as fiducials. Examples of commonly used tag-based optical tracking systems are Naviswiss ClipOn^{™}, ClaroNav MicronTracker ^{™}.

### Markers

Markers 20 - also called rigid bodies in the literature - are comprise several Fiducials 21 rigidly fixed altogether. The position of the Fiducial with respect to the Attachment Mechanism 22 or the tooltip is called the geometry and should be well known. During the tracking process, these fiducials 21 are detected, identified and their 3D position computed. If at least three fiducials are detected, an algorithm is further used (e. g. "Least-Squares Fitting of Two 3-D Point Sets", Arun 1987) to calculate the pose (position + orientation) of the Marker in the tracking system referential.

In IR systems, Fiducials 21 may be IR-LEDs or spheres/disks composed of reflective materials. Markers could alternatively be tags of known patterns. In this case, Fiducials are specific points on these patterns. With such a technology, a Marker could be defined with a single pattern.

The Attachment Mechanism 21 is used to fix/glue the Marker 20 both on the patient (e.g. through a pin) and the surgical instruments (e.g. digitizer used for registration, drill, ultrasound probe, or other tools).

As Markers are operating in the surgical field, they need to be sterile.

Reflective Markers have a typical base of carbon, stainless steel or titanium. Reflective disks or spheres are screwed on the base. Position of reflective fiducials is designed to provide a unique geometry to simplify their identification.

Active Markers comprise IR-LEDs. In a non-synchronized configuration, IR-LEDs are always on and should provide similar characteristics as the reflective Makers. If the IR-LEDs are synchronized with the tracking system (e. g. by mean of optical communication), they can emit more energy. Overall power consumption is also reduced and the battery lasts longer. This synchronization of the markers can alternatively be done via a wireless deterministic communication means previously described.

Having wireless Communication Means 30 between the Markers and the Optical Tracking System(s) allows to retrieve extra information from the Marker 20 (e. g. marker serial number, calibration, button status, battery status, or any other embedded sensor like inertial, gyroscopic, accelerometer) back to the Tracking System (see Figure 7) or direct to the Tablet (see Figure 8). In the other direction, overall application status could be displayed on the Marker 20 (e. g. status of the tracking on a visible LEDs, screen to display application info, targeting mechanism to keep the instrument on a trajectory or to reach an ablation area as illustrated in figure 11, error feedback, etc.). Depending on the application, the components may exchange data in another workflow. Markers could communicate between themselves independently to exchange data, retrieve warning/error status, etc. Even markers 20 not localized in the field of the optical tracker 10 could communicate and be identified by tracked markers20 . Their identification can be signalled to the tracker 10 permitting the end-user application on the tablet 40 to localize all activated markers 20, even ones not visible by the optical tracker 10.

As discussed previously, a deterministic wireless packet transmission mechanism is required in order to retrieve the timestamp from the optical tracking system or to send the real-time marker sensor data to the optical tracking system.

Tags markers are usually printed patterns placed on a flat surface and providing an attachment mechanism. ClaroNav^{™} and Naviswiss^{™} are providing system using such markers.

### Communication Means

Communication Means 30 enable the Tracking System 10 to send metrological data to the PC / Processing Unit 41. In a traditional system, communication means are wired. As such, there are no real needs to optimize the bandwidth. Raw data (e. g. full images) may be transferred to the PC for further processing. Wired connections on nomad or smaller Tracking Systems might however be a major concern within a surgical field. Accessibility of all the working field is of main importance for the surgeon. Wired accessories (e.g. Markers) are less and less tolerated in the sterile field for security reasons and ergonomics. They now are more and more replaced by wireless accessories.

The proposed system aims to reduce the data to a minimum to be able to send them via a wireless link like WPAN or Wi-Fi. As such, most of the tracking processing should be realized on the tracking system itself. In a minimal configuration only the timestamp (resp. counter), the pose (6 DoF) and the ID of every visible Markers 20 need to be transferred. Another advantage of this compact data transfer is to allow higher update rates as well as smaller latencies. Energy consumption will also be reduced if the data bandwidth is low. Saving energy is typically necessary if the tracking system is operated with a battery.

### Tablet

The Tablet 40 runs the Navigation Software. Even if this application is specific to a surgical gesture, Navigation Software share more or less the same basis. Marker poses or positions and/or orientations coming from the Optical Tracking System 10 through Communication Means 30 are retrieved/computed by the application running on the Tablet. Relative transformation of Markers 20 are further used to provide metrological information to the surgeon (e.g. real-time location of a biopsy needle in pre-operative imaging, cutting planes that should follow a navigated saw as defined in a pre-operative step, testing the range of motion of a joint, etc.).

As the Tablet 40 is no longer fixed on a Navigation Cart 50 and thanks to its compact size, it may be placed closer to or even in the sterile field. It should preferably be wrapped in a sterile bag so that the surgeon can touch it with his/her glows without being contaminated. Tablets could be any Tablet available on the market like an iPad^{™}, an Android Tablet ^{™} or a Microsoft Surface^{™}. It can also be a laptop with touch capabilities like a Lenovo Yoga^{™}. The tablet is preferably a medical-grade product.

It is to be noted that in an embodiment of the disclosure, the Tablet could be avoided. In this case, the Navigation Software is running in the Optical Tracking System. The interface is no longer the tablet and can be directly integrated in the instruments. For example, buttons on the markers could be used as input devices, screens and/or LEDs on the Markers could display navigation information.

### Other Embodiments of the Disclosure

In an embodiment of the disclosure, part of the processing is preferably done in the same housing as the tracking system in order to reduce the optical pose or position and/or orientation data bandwidth. For example, a processing unit located in the Optical Tracking System 10 can process the raw data coming from the Optical Sensors 11 up to the triangulation of the 3D position of the fiducials 21 and/or the pose or position and/or orientation of the Markers 20. A minimal wireless communication transfer packet would comprise, timestamp (respectively counter), poses (6 DoF) and IDs of the markers. This lightweight data transfer enables a higher update rate of the metrological data and by the way to reduce the overall latency of the system. This will furthermore reduce the energy consumption for the transmission. As an example, imagine a stereo tracking system composed of two VGA optical sensors (640 x 480 pixels). Pixels are grayscale 8 bits. If the raw data are transferred non-compressed to the Tablet, a bandwidth of 2 x 480 x 640 = 614'400 bytes = 4.9 mbits is necessary. If data are transferred through Bluetooth v1.2 with a throughput of about 80 kbit/s, about 4'900 / 80 s = 1 minute is necessary to get the next measurement. In the other hand if only the poses or position and/or orientation of the markers are communicated and two markers are visible in the images, a total of 2 x 6 floating values have to be transmitted. In case of 32 bits floating values, two poses (position and/or orientation) are stored in 2 × 6 × 4 = 48 bytes = 0. 375 kbit meaning that more than 200 update can be realized every second. For tracking system with limited processing power, the computation and transmission of marker poses (6 DoF) within a tight time constraint could be a limitation. In that case, required minimal information such as fiducials centroids in the images are transmitted to the tablet to compute markers poses (positions and/or orientations). The minimum data to send is deduced using the tracking system processing limit, communication bandwidth, tablet processing limitation, required maximal latency, required frame acquisition rate, and/or end-user final application refreshment rate.

In an embodiment of the disclosure, the Communication Means 30 are wireless (preferably WPAN), so that the tracking system 10 can be integrated in the infrastructure of the operating room requiring only a power source to operate. No data cables have to be installed in the room. Power for the Optical Tracking System 10 could be taken directly in the surgical lamp 60 and the device can directly be clamped on the Surgical Lamp 60 (see Figure 3). An alternate solution would be to have a battery pack attached to the tracking system 10 in a way that it is easily exchanged or recharged. The fixation mechanism may be a clip, a hook, a magnet, etc. The battery pack may be connected to the tracking system 10 via a standard USB connector like a USB charger.

In an embodiment of the disclosure, the Optical Tracking system 10 is preferably operating on batteries and is no longer fixed on the Navigation Cart but on another location, such as:
- Directly on a pole or an arm fixed on the operation table;
- Clamped on the separation between the anaesthetist and the surgical field;
- Placed on a mechanical arm that is part of a cluster of hanging surgical lamps;
- Fixed / clipped / integrated in other surgical equipment (C-Arm, surgical microscope, interventional MRI, CT scan, endoscope), fixed directly on the patient or on surgical instruments (e.g. on drills, saw, etc.).

In an embodiment of the disclosure, the Marker 20 has wireless communication means 30 (preferably WPAN, and/or an unidirectional/bidirectional optical channel). Communication can take place between Markers 20 themselves and/or between Markers 20 and Optical Tracking System(s) 10 and/or between Markers 20 and the Tablet 40 and/or between Optical Tracking System(s) 10 and the Tablet 40.

In an embodiment of the disclosure, wireless communication between the Optical Tracking System(s) 10 and active Markers 20 is preferably deterministic and enables to synchronize the overall system by either deciding the exact moment when the IR-LEDs 21 of the Markers 20 should be switched on and/or to synchronize the clocks of the different elements in order to define time slots. Using several Optical Tracking Systems 10 with such synchronization enable to timeslot their acquisition so that there is no cross-talk between them and the measurement accuracy is guaranteed. Having multiple Optical Tracking Systems 10 in parallel enable to increase the working volume by covering it with multiple cameras 11, reduce the line-of-sight problem as Markers 20 can be seen at different viewing angles, and/or increase the acquisition speed as the Optical Tracking Systems 10 are acquiring the ones after the others. For example, a system having an update rate of 25 Hz (40 ms) usually acquired the images of the fiducials within 1ms. Theoretically forty well synchronized Optical Tracking Systems 10 could acquire data keeping their 25 Hz update rate. The tablet 40 will effectively receive 1.000 measurement per seconds successively from the forty tracking systems 10.

In an embodiment of the disclosure, several Optical Tracking Systems 10 are wirelessly connected to the Tablet 40, redundant tracking data can be retrieved and used on the fly to verify the accuracy of measures. They alternatively can be used for optical data fusion.

In an embodiment of the disclosure, Optical Tracking Systems 10 may communicate between themselves independently to exchange data such as calibration, shocks issues. Connected Optical Tracking System(s) 10 may report issues of surrounding trackers to the tablet.

In an embodiment of the disclosure, wireless communication 30 enables to send extra information to the Optical Tracking System 10 (respectively to the Tablet 40). Such information could be the calibration of the Marker 20 (position of the Fiducials 21), button status, battery status, and/or other integrated memory or embedded sensor data (gyroscope, inertial, acceleration, temperature, humidity, GPS, temperature, blood pressure, electrodes, ECG, stimulators, etc.). In case of wireless deterministic communication (e.g. optical or near-infrared communication), integrated sensor data (e. g. inertial, gyroscopes) may be combined with optical measurement (pose of the Marker 20) to provide pose (position and/or orientation) information event if the Marker 20 is not or partly viewed by the Optical Tracking System(s) 10. This redundancy can alternatively be used to improve the accuracy by performing sensor fusion and/or as a redundant pose (position and/or orientation) information. The drift of an inertial sensor can be also corrected when the Marker 20 is in view of the Optical Tracking System 10. Figures 7 and 8 presents two different configurations where the time can be synchronised between Markers 20 and Optical Tracking 10 system in order to perform optimal sensor fusion.

In an embodiment of the disclosure, application data (respectively hi-level measurement data like estimation of registration error, reaching point information) may be sent back from the Tablet 40 (respectively Tracking System(s) 10) to the Markers 20. Actuators on the Markers 20 can be used to sense / display this information. For example, a digitizer can vibrate when an anatomical point is correctly registered, visible LEDs can display the status of the Marker 20 (e. g. colour range from green to red, blinking red, status can be the registration error, information if the marker is visible by a tracking system, etc.), a screen located on the Marker 20 can display application data, a visual targeting system on a tracked tool can help the surgeon to position it within a pre-determined trajectory, a sound can be emitted from a Marker 20, etc. The tablet (respectively Optical Tracking System(s)) could also refine, recalibrate, deactivate specific sensors on markers based on redundant information analysed on the fly.

In an embodiment of the disclosure as presented in Figure 11, the Marker 20 may comprise actuators --- (e.g. buttons 28, touchpads, and/or pressure sensors) and/or optionally displays (LEDs 26a, LCD 26band/or screen) in order for the surgeon to keep sterile when interacting with the surgical application that could run on the Tablet 40. In this case, the Tablet 40 has no need to be sterile. In extreme setup as the one of figure 11, there is no need of a tablet. The interface is directly on the probe hold by the surgeon.

In an embodiment of the disclosure, an "Intelligent Tool" or robot sharing the same deterministic wireless communication may directly use pose (position and/or orientation) data and/or other data coming from the different connected elements to perform a specific task. For example, the tool can stop drilling if a specific region is reached, the region being defined (pre-operatively) on base of scanner/MRI/ultrasound images. Another example is a robot that could receive the transformation between the end-effector and the patient and use it to operate (e.g. to drill a hole, to perform a biopsy, to move an endoscope to follow in real-time the movements of the patient, etc.).

In an embodiment of the disclosure, Optical Tracking Data may be broadcasted. Several devices may be connected for processing tracking data. They can independently process these data on different applications.

In an embodiment of the disclosure, a battery may be used to get rid of a power wire. In this case, the Optical Tracking System may be totally wireless. The battery may be a rechargeable one (via a wire, induction, and/or solar cells). The battery may be a supercapacitor and/or an accumulator. The battery may be permanently inside the same housing as the tracking device or clipped, so that it may easily be exchanged.

In an embodiment of the disclosure, the Optical Tracking System may be used for tracking purposes as well as to record image/videos during operation. Data can be 2D or 3D. For example, it is easy to compute disparity images/videos from a stereo camera 11. These recordings could be realized for archiving, reglementary and/or display purposes. Real-time disparity images/videos may be further used:
- To make any 3D computation on the reconstructed surface (e.g. rigid registration between pre-operative images and the reconstructed surface, gait analysis, measurement on the mesh, etc.);
- As an interface to the system in a similar way as the Microsoft Kinect^{™} is used for games. For example, the surgeon may trigger action by a specific hand/finger gestures in front of the tracking system. It may be used to start/stop recording. It may alternatively be used directly by a patient to interact with an interface while tracking in a rehabilitation application.
- For post-surgery analysis. Images/videos combined with any other internal/external metadata may be overlaid for post-surgery analysis (see Figure 6). Internal meta-data may be tracking parameters, errors in the system (triangulation, registration, etc.). External metadata may be vital parameters of the patient (e.g. heart rate, electrocardiogram, 3d reconstructions of the organs of the patient, etc.). Metadata may be augmented in the images like placing precisely the 3d reconstruction of an organ at the correct position in the image. Registration error or tool-tip of a marker may be overlaid at the correct location in an image. Note that most of these processing may alternatively be computed in real-time during the surgery.

As the Optical Tracking System may be very compact and mobile, it may be placed within the surgical field. In this case, the system may be either sterile or placed in a sterile drape. If no care is taken when selecting the drape, overall tracking accuracy can be hardly affected. In an embodiment of the disclosure, the Optical Tracking System comprises a mechanism to precisely fix a sterile window in front of the Optical Sensors (see Figure 4). The sterile window is integrated in the sterile drape that covers partly or entirely the Optical Tracking System. The sterile window and/or the sterile drape has a fixation mechanism (e. h. hooks, notches, holes, clips, magnets, etc.) to precisely fix the optical window on a complementary part located on the tracking system. Ideally the window should fit parallel to the optical sensors at a known distance. This window (especially the reflection within it) should be considered in the optical model for triangulation and during the calibration procedure in order to improve its overall metrological accuracy. This window is typically an optical-grade transparent thin plastic or glass. It can be composed of one part covering all the Optical Sensors or one window per Sensor. If a reproducible drape is not possible, it should be designed such that it affects as less as possible the optical measure.

In an embodiment of the disclosure, the Optical Tracking System comprises a Real-Time Supervision Unit (RTSU) that is operating all the time (e. g. when the system is shipped, used, moved and/or stored). The RTSU is sleeping until a shock and/or vibrations and/or a temperature and/or a hygrometry threshold is reached. If such an event occurs, the RTSU records it, optionally tags it with a global time. Such events can be retrieved by the driver/firmware. The system can decide to enter an error mode if specific events occur (typically when a big shock is detected the system can be decalibrated). This error mode can be left if a specific verification procedure is realized. The RTSU may be powered on an extra battery/accumulator so that it is operational even if the system is switched off. The RTSU accumulator could recharge when the tracking system is in operation and powered with another source.

In an embodiment of the disclosure, the system can be used in another field than surgery like interventional radiology, diagnostics, rehabilitation, reverse engineering, part checking and/or motion capture.

In an embodiment of the disclosure, fiducials and/or markers are placed on the person/object to be tracked, the optical tracking system is placed on a (autonomous) mobile platform which is following the person/object. The mobile platform can be a robotic arm, a cart, a (flying) drone, etc. Information from the mobile platform and the optical system can be combined to provide an absolute measurement data of the person/object. Information on the optical tracking system can be used to control the mobile platform. The real-time deterministic wireless packet transmission means can be used to achieve such a task.

### Operation of a Preferred Embodiment

The Optical Tracking System 10 acquires data from the Fiducials 21 through the Optical Sensors 11 during the navigated part of the surgery. At the start of each acquisition cycle, the tracking system is either sending a message to the active Markers 20 to switch their IR-LEDs 21 on, and/or flashing the IR-LEDs 21 around the optical sensors to reflect light in case of reflective Markers 20, and/or simply acquire a pair of images if the Markers 20 are Tags. For each Optical Sensor 11, angular position of the Fiducials 21 is further determined. Given epipolar and marker geometry constraints, the fiducials 21 are identified and triangulated. At the end, their 3D position and the Marker 20 they belong to is known. A rigid registration algorithm is further used to compute the poses (positions and/or orientations) of the Markers 20 with respect to the Optical Tracking System 10 referential. Timestamp, markers IDs and their respective poses i.e. positions and/or orientations (and other optional data) are sent to the Tablet 40 though a wireless link 30. The wireless data transfer protocol is designed to optimize the bandwidth in order to guarantee the best update rate and the minimum latency. The application running on the Tablet 40 uses poses (position and/or orientation) information to assist the surgeon during the surgery as an illustrative application of the disclosure.

Alternatively, application information can be pushed back (directly or via the tracking system) to the markers 20 in order to provide a visual or tactile feedback on the tracked instruments used by the surgeon.

Markers 20 may integrate embedded sensors. A deterministic wireless packet transmission protocol is used to transmit embedded sensors data to the Optical Tracking System 10 while optical tracking is in operation. As the system is deterministic, it is possible to synchronize the timestamp of both marker sensor data with their respective pose (position and/or orientation) data. When the timestamps are expressed in the same clock referential, it is possible to adjust any of the data to fit an exact same timestamp using either interpolation or extrapolation techniques. Synchronized data can be further used for data fusion. The sensed data as well as raw data expressed in the same clock referential can further be transmitted to the Navigation PC or Tablet PC 40.

## Claims

1. An optical tracking system for medical applications, with an optical tracking unit (10) comprising at least one sensor (11), a processing unit and energy providing means (12), the optical tracking unit, the sensor and the processing unit being integrated in a single housing, said system further comprising
at least one marker (20) to be attached to an object or to a person; wherein said at least one marker (20) comprises at least one embedded sensor configured to provide embedded sensor data;
separate or remote display means (40) receiving data at least from said tracking unit;
wireless communication means (30) connecting at least said tracking unit (10) with said display means (40) and allowing data to be transmitted between said tracking unit (10) and said display means (40),
wherein said sensor (11) is configured to detect said marker and generate sensor data,
wherein the processing unit is configured to generate position and/or orientation data of said marker relatively to said tracking unit by data processing the sensor data, and
wherein the wireless communication means is configured to transmit the position and/or orientation data to the display means such that data transmission between the tracking unit and the display means is optimized to a communication channel of the wireless communication means (30),
wherein said at least one marker comprises fiducials comprising light generating elements, wherein the light generating elements are both used to transmit the embedded sensor data and used as fiducials during a sensing phase.

2. The optical tracking system as defined in claim 1, wherein said display means is a tablet computer.

3. The optical tracking system as defined in one of the preceding claims, wherein the energy providing means (12) is a battery pack, wherein the battery pack is integrated in the same housing as the optical tracking unit (10).

4. The optical tracking system as defined in one of the preceding claims, wherein the energy providing means (12) is a battery pack and wherein the battery pack is attached to the housing and can easily be removed and/or replaced.

5. The optical tracking system as defined in one of the preceding claims, wherein said unit comprises at least two sensors.

6. The optical tracking system as defined in claim 5, wherein the 3D position of said marker can be computed using a triangulation technique.

7. The optical tracking system as defined in the preceding claim, wherein at least one of said sensor is a camera.

8. The optical tracking system as defined in one of the preceding claims, wherein the tracking unit comprises attachment means (14) to attach a surgical drape (60).

9. The optical tracking system as defined in one of the preceding claims, wherein said wireless communication means use IEEE 802.11 standard such as Wi-Fi, or IEEE 802.15 standard such as Bluetooth.

10. The optical tracking system as defined in one of the preceding claims 1 to 9 wherein said system is portable.

11. A surgical device combined with an optical tracking system as defined in one of the preceding claims 1 to 9.

## Patentansprüche

1. Optisches Verfolgungssystem für medizinische Anwendungen, mit einer optischen Verfolgungseinheit (10), umfassend wenigstens einen Sensor (11), eine Verarbeitungseinheit und Energiebereitstellungsmittel (12), wobei die optische Verfolgungseinheit, der Sensor und die Verarbeitungseinheit in einem einzelnen Gehäuse integriert sind, wobei das System ferner umfasst:
wenigstens einen Marker (20), der an einem Objekt oder einer Person befestigt werden soll; wobei der wenigstens eine Marker (20) wenigstens einen eingebetteten Sensor umfasst, der dafür ausgelegt ist, eingebettete Sensordaten bereitzustellen;
separates oder entfernt angeordnetes Anzeigemittel (40), das Daten wenigstens von der Verfolgungseinheit empfängt; drahtloses Kommunikationsmittel (30), das wenigstens die Verfolgungseinheit (10) mit dem Anzeigemittel (40) verbindet und es Daten erlaubt, zwischen der Verfolgungseinheit (10) und dem Anzeigemittel (40) übertragen zu werden,
wobei der Sensor (11) dafür ausgelegt ist, den Marker zu detektieren und Sensordaten zu generieren,
wobei die Verarbeitungseinheit dafür ausgelegt ist, durch Datenverarbeitung der Sensordaten, Positions- und/oder Orientierungsdaten des Markers relativ zu der Verfolgungseinheit zu generieren, und
wobei das drahtlose Kommunikationsmittel dafür ausgelegt ist, die Positions- und/oder Orientierungsdaten an das Anzeigemittel zu übertragen, sodass eine Datenübertragung zwischen der Verfolgungseinheit und der Anzeigemittel auf einen Kommunikationskanal des drahtlosen Kommunikationsmittels (30) optimiert ist,
wobei der wenigstens eine Marker Referenzmarken (Fiducials) umfasst, die lichtgenerierende Elemente umfassen, wobei die lichtgenerierenden Elemente verwendet werden, um die eingebetteten Sensordaten zu übertragen, und während einer Erkennungsphase auch als Referenzmarken verwendet werden.

2. Optisches Verfolgungssystem wie in Anspruch 1 definiert, wobei das Anzeigemittel ein Tablet-Computer ist.

3. Optisches Verfolgungssystem wie in einem der vorstehenden Ansprüche definiert, wobei das Energiebereitstellungsmittel (12) ein Batteriesatz ist, wobei der Batteriesatz in demselben Gehäuse integriert ist wie die optische Verfolgungseinheit (10).

4. Optisches Verfolgungssystem wie in einem der vorstehenden Ansprüche definiert, wobei das Energiebereitstellungsmittel (12) ein Batteriesatz ist und wobei der Batteriesatz an dem Gehäuse befestigt ist und leicht entfernt und/oder ersetzt werden kann.

5. Optisches Verfolgungssystem wie in einem der vorstehenden Ansprüche definiert, wobei die Einheit wenigstens zwei Sensoren umfasst.

6. Optisches Verfolgungssystem wie in Anspruch 5 definiert, wobei die 3D-Position des Markers unter Verwendung eines Triangulationsverfahrens berechnet werden kann.

7. Optisches Verfolgungssystem wie im vorstehenden Anspruch definiert, wobei wenigstens einer der Sensoren eine Kamera ist.

8. Optisches Verfolgungssystem wie in einem der vorstehenden Ansprüche definiert, wobei die Verfolgungseinheit Befestigungsmittel (14) umfasst, um ein Operationstuch (60) zu befestigen.

9. Optisches Verfolgungssystem wie in einem der vorstehenden Ansprüche definiert, wobei das drahtlose Kommunikationsmittel einen IEEE 802.11-Standard wie etwa Wi-Fi oder einen IEEE 802.15-Standard wie etwa Bluetooth nutzt.

10. Optisches Verfolgungssystem wie in einem der vorstehenden Ansprüche 1 bis 9 definiert, wobei das System tragbar ist.

11. Chirurgische Vorrichtung in Kombination mit einem optischen Verfolgungssystem wie in einem der vorstehenden Ansprüche 1 bis 9 definiert.

## Revendications

1. Système de suivi optique pour applications médicales, avec une unité de suivi optique (10) comprenant au moins un capteur (11), une unité de traitement et un moyen de fourniture d'énergie (12), l'unité de suivi optique, le capteur et l'unité de traitement étant intégrés dans un seul boîtier, ledit système comprenant en outre :
au moins un marqueur (20) destiné à être fixé à un objet ou à une personne ; ledit au moins un marqueur (20) comprenant au moins un capteur intégré configuré pour fournir des données de capteur intégrées ;
un moyen d'affichage séparé ou à distance (40) recevant des données au moins de ladite unité de suivi ;
un moyen de communication sans fil (30) reliant au moins ladite unité de suivi (10) audit moyen d'affichage (40) et permettant aux données d'être transmises entre ladite unité de suivi (10) et ledit moyen d'affichage (40),
ledit capteur (11) étant configuré pour détecter ledit marqueur et générer des données de capteur,
l'unité de traitement étant configurée pour générer des données de position et/ou d'orientation dudit marqueur par rapport à ladite unité de suivi en traitant les données de capteur, et
le moyen de communication sans fil étant configuré pour transmettre les données de position et/ou d'orientation au moyen d'affichage de sorte que la transmission des données entre l'unité de suivi et le moyen d'affichage soit optimisée sur un canal de communication du moyen de communication sans fil (30),
ledit au moins un marqueur comprenant des repères constitués d'éléments générateurs de lumière, les éléments générateurs de lumière étant à la fois utilisés pour transmettre les données de capteur intégré et utilisés comme repères au cours d'une phase de détection.

2. Système de suivi optique selon la revendication 1, ledit moyen d'affichage étant un ordinateur tablette.

3. Système de suivi optique selon l'une quelconque des revendications précédentes, le moyen de fourniture d'énergie (12) étant un bloc-batterie, le bloc-batterie étant intégré dans le même boîtier que l'unité de suivi optique (10).

4. Système de suivi optique selon l'une quelconque des revendications précédentes, le moyen de fourniture d'énergie (12) étant un bloc-batterie et le bloc-batterie étant fixé au boîtier et pouvant être facilement retiré et/ou remplacé.

5. Système de suivi optique selon l'une quelconque des revendications précédentes, ladite unité comprenant au moins deux capteurs.

6. Système de suivi optique selon la revendication 5, la position 3D dudit marqueur pouvant être calculée à l'aide d'une technique de triangulation.

7. Système de suivi optique selon la revendication précédente, au moins l'un des capteurs étant une caméra.

8. Système de suivi optique selon l'une quelconque des revendications précédentes, l'unité de suivi comprenant des moyens de fixation (14) pour fixer un drap chirurgical (60).

9. Système de suivi optique selon l'une quelconque des revendications précédentes, ledit moyen de communication sans fil utilisant la norme IEEE 802.11, telle que Wi-Fi, ou la norme IEEE 802.15, telle que Bluetooth.

10. Système de suivi optique selon l'une quelconque des revendications précédentes 1 à 9, ledit système étant portable.

11. Dispositif chirurgical associé à un système de suivi optique selon l'une quelconque des revendications précédentes 1 à 9.
